# EUROPEAN PATENT SPECIFICATION

(11) **EP 1 922 059 B1**
(45) Date of publication and mention of the grant of the patent: **22.06.2016**
(21) Application number: 06802301.9
(22) Date of filing: 24.08.2006
(51) Int. Cl.: A61K 9/08, A61K 31/4535

(54) **STABILIZED AND PRESERVED KETOTIFEN OPHTHALMIC COMPOSITIONS**
STABILISIERTE UND ERHALTENE KETOTIFEN-AUGENZUSAMMENSETZUNGEN
COMPOSITIONS OPHTALMIQUES DE KETOTIFENE STABILISEES CONTENANT UN AGENT DE CONSERVATION

(30) Priority: 26.08.2005 US 212959
(43) Date of publication of application: 21.05.2008
(60) Divisional application: 10177749.8
(73) Proprietor: Novartis AG, 4056 Basel (CH); Novartis Pharma GmbH, 1230 Wien (AT)
(72) Inventor: TSAO, Fu-Pao, Lawrenceville, GA 30043 (US); WONG, Michelle, P., San Francisco, CA 94132 (US); YEN, Shau, Fong, Livingston, NJ 07039 (US)
(74) Representative: Barbier, Denis Robert
(86) International application number: PCT/US2006/033161
(87) International publication number: WO 2007/025092

(56) References cited:
- EP-A- 0 354 186
- WO-A-03/059069
- US-A1- 2002 127 281
- US-A1- 2002 165 254

## Description

The present invention relates to ophthalmic compositions comprising ketotifen as a pharmaceutically active agent and a source of hydrogen peroxide as a preservative for use in treating allergic conjunctivitis. U.S. Patent Nos. 5,725,887 (the '887 patent) and 5,607,698, and Application Serial No. 11/078,209, disclose and claim methods for the preservation of ophthalmic solutions using stabilized hydrogen peroxide and compositions so preserved.

WO 03/059069 relates to a method of preserving ophthalmic solutions with trace amounts of stabilized peroxy compounds and alkaline earth metal salts against the growth of mould. US 2002/165254 discloses autoclavable ophthalmic compositions comprising an ophthalmic drug selected from ketotifen and dexamethasone and a method for stabilizing such compositions.

However, hydrogen peroxide is a strong oxidation agent. Many chemical or drug substances are not compatible with hydrogen peroxide, i.e., are susceptible to chemical oxidation by hydrogen peroxide. Ketotifen is a pharmaceutically active agent susceptible to chemical oxidation by hydrogen peroxide. While ketotifen is not compatible with low concentrations of hydrogen peroxide when formulated at neutral pH, i.e., a pH of about 7, it has now been found that ketotifen is compatible with low concentrations of hydrogen peroxide when formulated at a pH lower than neutral pH.

### Summary of the Invention

In one aspect, an ophthalmic composition is provided which comprises:
(a) a ketotifen salt, wherein the concentration of ketotifen salt as ketotifen is from 0.01 to 0.1 % (w/v);
(b) a hydrogen peroxide source providing hydrogen peroxide in a trace amount of from 0.001 to 0.1 % (w/v);
(c) one or more ocularly compatible hydrogen peroxide stabilizers, wherein the composition is at a pH from 4 to 5.3, sufficient to stabilize the ketotifen salt against oxidation by hydrogen peroxide.

In another aspect, a method for the treatment and prevention of allergic conjunctivitis is provided, which comprises topically administering to a subject suffering from or susceptible to the allergic conjunctivitis an effective amount of said ophthalmic composition.

### Detailed Description of the Invention

The present invention is directed to stable ophthalmic compositions comprising a ketotifen salt, a hydrogen peroxide source providing hydrogen peroxide in an amount of from about 0.001 to about 0.1% weight/volume (w/v) and one or more ocularly compatible hydrogen peroxide stabilizers. The ophthalmic compositions are formulated at a pH from 4 to 5.3, to stabilize the ketotifen salt against oxidation by hydrogen peroxide. Thus, the acid form of ketotifen is more stable than the neutral form of ketotifen.

The ketotifen salt is, e.g., ketotifen hydrochloride, ketotifen hydrobromide, ketotifen pamoate, ketotifen maleate, ketotifen sulfate and ketotifen fumarate. A preferred ketotifen salt is ketotifen fumarate. The concentration of ketotifen salt as ketotifen is typically from 0.01 to 0.1% (w/v) and preferably from 0.02 to 0.06% (w/v).

Trace amounts of peroxy compounds stabilized with a hydrogen peroxide stabilizer, especially diethylene triamine penta(methylene phosphonic acid) or 1-hydroxyethylidene-1,1-diphosphonic acid may be utilized as a preservative for the ophthalmic compositions of the invention. A hydrogen peroxide source is any peroxy compound that is hydrolyzed in water to produce hydrogen peroxide. Examples of hydrogen peroxide sources, which provide an effective resultant amount of hydrogen peroxide, include sodium perborate, sodium perborate tetrahydrate, sodium peroxide and urea peroxide. It has been found that peracetic acid, an organic peroxy compound, cannot be stabilized utilizing the present system.

The hydrogen peroxide source is present in an amount sufficient to provide from 0.001 to 0.1% (w/v) hydrogen peroxide, and preferably from 0.001 to 0.01% (w/v) hydrogen peroxide. As an example, the hydrogen peroxide source, sodium perborate tetrahydrate, can be present in an amount of from about 0.005 to about 0.05%(w/v).

A hydrogen peroxide stabilizer, as used herein, means any of the known stabilizers of peroxy compounds including phosphonates, phosphates, stannates, etc. Physiologically compatible salts of phosphonic acids may also be used, such as diethylene triamine penta(methylene-phosphonic acid) and physiologically compatible salts thereof and 1-hydroxyethylene-1,1,-diphosphonic acid and physiologically acceptable salts thereof. Other stabilizers of peroxy compounds useful in the practice of the present invention are disclosed in the '887 patent, *inter alia,* column 5, line 55 to column 6, line 34.

The above stabilizers can be used in almost all indications described below. However, when the solution is to come in contact with a hydrogel soft contact lens, stannate stabilizers are to be avoided as they tend to "cloud" the lens material.

When the peroxy stabilizer is diethylene triamine penta(methylene-phosphonic acid), it can be present in the composition in an amount from 0.001 to 0.02 % (w/v) or in an amount from 0.002 to 0.012 % (w/v).

When the peroxy stabilizer is 1-hydroxyethylene-1,1,-diphosphonic acid it can be present in the composition in an amount from 0.002 to 0.2 % (w/v).

Stabilizers other than diethylene triamine penta(methylene-phosphonic acid (sold by Monsanto Company, St. Louis, Mo., under the trademark DEQUEST 2060) and physiologically compatible salts thereof and 1-hydroxyethylene-1,1,-diphosphonic acid and physiologically acceptable salts thereof are employed in physiologically tolerable amounts.

Soluble alkaline earth metal salts can be used in the ophthalmic compositions in amounts from about 0.002 to about 0.2% (w/v) of the preserved solution, or from about 0.01% to about 0.1 % (w/v) of the preserved solution. Water soluble salts of magnesium and calcium are such alkaline earth metal salts. Addition of such soluble alkaline earth metal salts increases antifungal preservative efficacy in ophthalmic compositions preserved with low amounts of hydrogen peroxide.

As stated above, the ophthalmic compositions of the present invention are formulated at a lower pH value than neutral pH which is sufficient to stabilize the ketotifen salt against oxidation by hydrogen peroxide, at a pH of from 4 to 5.3. As an example, the ophthalmic composition is formulated at a pH of from 4 to 5.3. The pH can be adjusted as desired by incorporation of suitable amounts of acid or base of a physiologically tolerable nature in the amounts employed, e.g., hydrochloric acid and sodium hydroxide.

There may be present in the ophthalmic compositions according to the present invention one or more conventional, substantially inert, physiologically acceptable tonicity enhancing agents. Suitable such agents include, e.g., mannitol, sorbitol, glycerol, alkali metal halides, phosphates, hydrogen phosphate and borates. Preferred are sodium chloride, sodium phosphate monobasic and sodium phosphate dibasic. The function of such tonicity enhancing agents is to assure approximate physiologic tonicity to the composition when instilled in the eye or to help assure such tonicity upon dilution if dilution is necessary prior to contact with the eye due to peroxide content as indicated above.

Preferably sufficient tonicity enhancing agents are present in the ophthalmic composition so that it is substantially isotonic or, such that, upon decomposition or dilution of the hydrogen peroxide therein, the resulting composition is substantially isotonic, e.g., substantially equivalent in tonicity to a 0.9% (w/v) of aqueous sodium chloride solution. Preferably, the amount of tonicity enhancing agent present in the ophthalmic composition is from about 0.01 to about 1 % (w/v).

The ophthalmic compositions of the present invention can also include one or more viscosity enhancing agents. Examples of viscosity enhancing agents include, but are not limited to, cellulosic ethers, such as hydroxypropyl methyl cellulose (HPMC), hydroxyethyl cellulose (HEC) and ethylhydroxyethyl cellulose; polyacrylic acid; polyvinylalcohol; polyvinyl pyrrolidone; alginates; carrageenans; guar, karaya, agarose, locust bean and xanthan gums.

As an example, an ophthalmic composition of the present invention comprises from about 0.0138 to about 0.138 % (w/v) of ketotifen fumarate, and about 0.005 to about 0.5% (w/v) of sodium perborate, about 0.001 to about 0.01 % (w/v) of diethylene triamine penta(methylene-phosphonic acid) and physiologically compatible salts thereof or about 0.002 to about 0.2% (w/v) of 1-hydroxyethylene-1,1,-diphosphonic acid and physiologically acceptable salts salts thereof, wherein the composition has a pH of from about 3.5 to about 6. Purified water is added to bring the total to 100%.

The ophthalmic compositions of the present invention are characterized by their extraordinary stability, even under accelerated conditions, for example by heating the solutions to 100°C for 24 hours. Thus, the shelf life of these compositions is enhanced. Moreover, the compositions of the invention are characterized by physiological tolerability subsequent to hydrogen peroxide decomposition.

Another advantage in using hydrogen peroxide in ophthalmic compositions is that the trace amount of hydrogen peroxide is destroyed once comes in contact with the eye. For example, catalase existing in the eye tissue will cause the breakdown of the hydrogen peroxide into water and oxygen. As a result, the ophthalmic composition, upon application, becomes preservative free and greatly minimizes adverse reactions. The problems associated with other preservatives, such as the inability to break down to innocuous compounds, are eliminated.

The ophthalmic compositions of the invention can be made in any conventional manner. For example, all of the components other than the hydrogen peroxide and water can be placed in a container and fresh, preferably concentrated, hydrogen peroxide added thereto with mixing. Alternatively the dry components can be rubbed up with a small portion of liquid stabilizer, then the remainder of the stabilizer added, followed by the hydrogen peroxide and most of the water. Other components, e.g., tonicity adjusting agents and viscosity enhancing agents can then be added or the formed composition can be added to such agents. One of ordinary skill in the art will be aware of numerous variations in the manner of formulating the compositions of the invention.

Another aspect of the present invention is directed to a method for the treatment and prevention of allergic conjunctivitis. The method comprises administering to a subject suffering from or susceptible to allergic conjunctivititis an effective amount of the aforementioned ophthalmic composition.

In one embodiment of this method, the ophthalmic compositions can be applied topically to the eye, preferably in the form of eye drops for the treatment and reduction in itching of the eye due to allergic conjunctivitis and for the treatment and reduction of signs or any symptoms of seasonal allergic conjunctivitis. Dosage of the ophthalmic compositions will depend on severity of the allergic conjunctivitis and the concentration of the ketotifen salt in the composition and can be readily determined by one skilled in the art. Typically, between 1 drop and 10 drops, or between 1 drop and 5 drops, or between 1 drop and 3 drops are administered at one time when employing the compositions of the present invention.

In yet another embodiment of this method, the ophthalmic compositions can also be formulated for use in contact lens care solutions, e.g., contact lens wetting, storing, lubricating, cleaning, disinfecting and cosmetic care solutions.The ophthalmic compositions of the present invention can be packaged in any pharmaceutically acceptable packaging, but it is desirable to package them in squeezable plastic multidose containers such as dropper bottles. Such bottles can be made, e.g., of polyethylene or polypropylene or mixtures thereof. A dropper bottle will typically dispense between about 12 µL and about 50 µL per drop. When it is desirable to "neutralize" the peroxide activity, by any means known, such as rinsing, contacting the solution with platinum, catalase or any other substance known to decompose hydrogen peroxide, will suffice. Additional physiological compatible peroxide neutralizing agents include reducing agent, such as pyruvic acid; and suitable salts thereof, such as the sodium salt.

The following examples are presented for illustrative purposes and are not intended to limit the scope of this invention, but to demonstrate the stability of the ophthalmic compositions as stabilized in accordance with the present invention. All parts are by %(w/v) unless otherwise indicated.

### Example 1

**Table 1. Ketotifen-Perborate Formulation**

| **Formulation** | **Ketotifen Fumarate (mg/mL)** | **pH** | **NaCl (mg/mL)** | **Na Perborate.4 H₂O(mg/mL)** | **Dequest (mg/mL)** | **BoricAcid (mg/mL)** | **NaBorate (mg/mL)** |
|---|---|---|---|---|---|---|---|
| Comparative formulation 1 | 0.345 | 6.3 | 6.65 | 0.27 | 0.06 | 5.0 | 0.05 |
| Comparative formulation 2 | 0 | 6.3 | 6.65 | 0.27 | 0.06 | 5.0 | 0.05 |
| Comparative formulation 3 | 0 | 5.3 | 6.65 | 0.27 | 0.06 | 5.0 | 0.05 |
| Formulation 4 | 0.345 | 5.3 | 6.65 | 0.2 | 0.06 | 5.0 | 0.05 |

The stability of the formulations shown in Table 1 are evaluated by filling the formulations in bottles and storing the formulations at 55°C for various times. The results are summarized in Table 2 below.

**Table 2. Ketotifen Fumarate Concentrations (mg/mL) of Samples Filled in Polypropylene Bottles and Stored at 55°C**

| **Formulation** | **Initial** | **1 Week at 55°C** | **2 Weeks at 55°C** | **4 Weeks at 55°C** |
|---|---|---|---|---|
| Comparative formulation 1 | 0.340 | 0.319 | 0.323 | 0.296 |
| Comparative formulation 2 | 0.00 | 0.00 | 0.00 | 0.00 |
| Comparative formulation 3 | 0.00 | 0.00 | 0.00 | 0.00 |
| Formulation 4 | 0.336 | 0.337 | 0.342 | 0.341 |

The formulations listed in Table 2 met USP preservative effectiveness test criteria as required by FDA for the preservation of ophthalmic solutions..

The results as shown in Table 2 indicate that ketotifen is stable under evaluated conditions, especially formulation 4, which is formulated at a lower pH than comparative formulation 1.

**Table 3. Ketotifen Perborate Formulations**

| | |
|---|---|
| Comparative formulation **1** | 0.025% Ketotifen (0.0345% ketotifen fumarate) |
| | 2% Propylene Glycol |
| | 0.3% HPMC (E4M) |
| | 0.006% Dequest 2060 |
| | 0.028% Sodium Perborate |
| | added Purified Water to the volume |
| | pH adjusted to 5.487 |
| | 296 mOsm/kg Tonicity |
| **Formulation 2** | 26.38 g of 10 bottles of Zaditor [0.025% ketotifen (0.0345% ketotifen fumarate, 2.5 mL)] put together |
| | 60 ppm Dequest 2060 |
| | 0.028% Sodium Perborate |
| | pH = 4.484 |

The stability of the formulations shown in Table 3 are evaluated by heating the formulations at 100°C for 24 hours. The results are summarized in Table 4 below.

**Table 4. Ketotifen Perborate Formulations Heated at 100°C for 24 Hours**

| | **Stability of Sodium** | | **pH** | |
|---|---|---|---|---|
| **Formulation** | **Perborate (hot,stability, %)** | **Stability of Ketotifen (%)** | Before Heated | After Heated |
| Comparative formulation 1 | 79.7 | 92.6 | 5.49 | 4.87 |
| Formulation 2 | 93.3 | 102.7 | 4.48 | 4.43 |

The results shown in Table 4 indicate that ketotifen is stable under the aforementioned accelerated conditions.

## Claims

1. An ophthalmic composition comprising:
(a) a ketotifen salt, wherein the concentration of ketotifen salt as ketotifen is from 0.01 to 0.1% (w/v);
(b) a hydrogen peroxide source providing hydrogen peroxide in a trace amount of from 0.001 to 0.1 % (w/v); and
(c) one or more ocularly compatible hydrogen peroxide stabilizers, wherein the composition is at a pH from 4 to 5.3.

2. The composition of Claim 1, wherein the ketotifen salt is ketotifen fumarate.

3. The composition of Claim 1, wherein the hydrogen peroxide source is selected from the group consisting of sodium perborate, sodium perborate tetrahydrate, sodium peroxide and urea peroxide.

4. The composition of Claim 1, wherein the hydrogen peroxide source provides hydrogen peroxide in an amount of from 0.001 to 0.01% (w/v).

5. The composition of Claim 1, wherein the one or more hydrogen peroxide stabilizers is selected from the group consisting of diethylene triamine penta(methylene phosphonic acid), 1-hydroxyethylidene-1,1-diphosphonic acid and physiologically compatible salts thereof.

6. The composition of Claim 1, wherein the hydrogen peroxide stabilizer is diethylene triamine penta(methylene phosphonic acid).

7. The composition of Claim 1, wherein hydrogen peroxide stabilizer is 1-hydroxyethylidene-1,1-diphosphonic acid.

8. The composition of Claim 6, wherein the composition comprises from 0.001 to 0.02% (w/v) of diethylene triamine penta(methylene phosphonic acid) or a physiologically compatible salt thereof.

9. The composition of Claim 7, wherein the composition comprises from 0.002 to 0.2% (w/v) of 1-hydroxyethylidene-1,1-diphosphonic acid or a physiologically compatible salt thereof.

10. The composition of Claim 1, wherein the composition further comprises a tonicity enhancing agent.

11. The composition of Claim 1 comprising:
(a) 0.02 to 0.06% (w/v) of a ketotifen salt as ketotifen;
(b) 0.001 to 0.01% (w/v) of hydrogen peroxide; and
(c) one or more ocularly compatible hydrogen peroxide stabilizers, wherein the composition is at a pH from 4 to 5.3.

12. An effective amount of an ophthalmic composition according to any preceding claim for topical administration to a subject suffering from or susceptible to allergic conjunctivitis for use in the treatment and prevention of allergic conjunctivitis.

## Patentansprüche

1. Ophthalmische Zusammensetzung, umfassend:
(a) ein Ketotifen-Salz, wobei die Konzentration von Ketotifen-Salz als Ketotifen 0,01 bis 0,1% (w/v) beträgt;
(b) eine Wasserstoffperoxidquelle, die Wasserstoffperoxid in einer Spurenmenge von 0,001 bis 0,1% (w/v) bereitstellt; und
(c) einen oder mehrere augenverträgliche Wasserstoffperoxidstabilisatoren, wobei die Zusammensetzung einen pH-Wert von 4 bis 5,3 aufweist.

2. Zusammensetzung nach Anspruch 1, wobei es sich bei dem Ketotifen-Salz um Ketotifenfumarat handelt.

3. Zusammensetzung nach Anspruch 1, wobei die Wasserstoffperoxidquelle aus der Gruppe bestehend aus Natriumperborat, Natriumperborattetrahydrat, Natriumperoxid und Harnstoffperoxid ausgewählt ist.

4. Zusammensetzung nach Anspruch 1, wobei die Wasserstoffperoxidquelle Wasserstoffperoxid in einer Menge von 0,001 bis 0,01% (w/v) bereitstellt.

5. Zusammensetzung nach Anspruch 1, wobei der bzw. die Wasserstoffperoxidstabilisatoren aus der Gruppe bestehend aus Diethylentriaminpenta(methylenphosphonsäure), 1-Hydroxyethyliden-1,1-diphosphonsäure und physiologisch verträglichen Salzen davon ausgewählt ist bzw. sind.

6. Zusammensetzung nach Anspruch 1, wobei es sich bei dem Wasserstoffperoxidstabilisator um Diethylentriaminpenta(methylenphosphonsäure) handelt.

7. Zusammensetzung nach Anspruch 1, wobei es sich bei dem Wasserstoffperoxidstabilisator um 1-Hydroxyethyliden-1,1-diphosphonsäure handelt.

8. Zusammensetzung nach Anspruch 6, wobei die Zusammensetzung 0,001 bis 0,02% (w/v) Diethylentriaminpenta(methylenphosphonsäure) oder ein physiologisch verträgliches Salz davon umfasst.

9. Zusammensetzung nach Anspruch 7, wobei die Zusammensetzung 0,002 bis 0,2% (w/v) 1-Hydroxyethyliden-1,1-diphosphonsäure oder ein physiologisch verträgliches Salz davon umfasst.

10. Zusammensetzung nach Anspruch 1, wobei die Zusammensetzung ferner einen Tonizitätsverstärker umfasst.

11. Zusammensetzung nach Anspruch 1, umfassend:
(a) 0,02 bis 0,06% (w/v) eines Ketotifen-Salzes als Ketotifen;
(b) 0,001 bis 0,01% (w/v) Wasserstoffperoxid und
(c) einen oder mehrere augenverträgliche Wasserstoffperoxidstabilisatoren, wobei die Zusammensetzung einen pH-Wert von 4 bis 5,3 aufweist.

12. Wirksame Menge einer ophthalmischen Zusammensetzung nach einem der vorhergehenden Ansprüche zur topischen Verabreichung an einen Empfänger, der an allergischer Konjunktivitis leidet oder dafür anfällig ist, zur Verwendung bei der Behandlung und Prävention von allergischer Konjunktivitis.

## Revendications

1. Composition ophtalmique comprenant :
(a) un sel de kétotifène, la concentration de sel de kétotifène exprimée en kétotifène étant de 0,01 à 0,1 % (m/v);
(b) une source de peroxyde d'hydrogène fournissant du peroxyde d'hydrogène en une quantité trace de 0,001 à 0,1 % (m/v) ; et
(c) un ou plusieurs stabilisants de peroxyde d'hydrogène oculairement compatibles, la composition étant à un pH de 4 à 5,3.

2. Composition de la revendication 1, dans laquelle le sel de kétotifène est le fumarate de kétotifène.

3. Composition de la revendication 1, dans laquelle la source de peroxyde d'hydrogène est choisie dans le groupe constitué des perborate de sodium, perborate de sodium tétrahydraté, peroxyde de sodium et peroxyde d'urée.

4. Composition de la revendication 1, dans laquelle la source de peroxyde d'hydrogène fournit du peroxyde d'hydrogène en une quantité de 0,001 à 0,01 % (m/v).

5. Composition de la revendication 1, dans laquelle les un ou plusieurs stabilisants de peroxyde d'hydrogène sont choisis dans le groupe constitué de l'acide diéthylènetriaminepenta(méthylènephosphonique), l'acide 1-hydroxyéthylidène-1,1-diphosphonique et des sels physiologiquement compatibles de ceux-ci.

6. Composition de la revendication 1, dans laquelle le stabilisant de peroxyde d'hydrogène est l'acide diéthylènetriaminepenta-(méthylènephosphonique).

7. Composition de la revendication 1, dans laquelle le stabilisant de peroxyde d'hydrogène est l'acide 1-hydroxyéthylidène-1,1-diphosphonique.

8. Composition de la revendication 6, **caractérisée en ce que** la composition comprend de 0,001 à 0,02 % (m/v) d'acide diéthylènetriaminepenta(méthylènephosphonique) ou un sel physiologiquement compatible de celui-ci.

9. Composition de la revendication 7, **caractérisée en ce que** la composition comprend de 0,002 à 0,2 % (m/v) d'acide 1-hydroxyéthylidène-1,1-diphosphonique ou un sel physiologiquement compatible de celui-ci.

10. Composition de la revendication 1, **caractérisée en ce que** la composition comprend en outre un agent augmentant la tonicité.

11. Composition de la revendication 1 comprenant :
(a) 0,02 à 0,06 % (m/v) d'un sel de kétotifène exprimé en kétotifène ;
(b) 0,001 à 0,01 % (m/v) de peroxyde d'hydrogène ; et
(c) un ou plusieurs stabilisants de peroxyde d'hydrogène oculairement compatibles, la composition étant à un pH de 4 à 5,3.

12. Quantité efficace d'une composition ophtalmique selon l'une quelconque des revendications précédentes pour administration topique à un sujet souffrant ou susceptible de conjonctivite allergique pour utilisation dans le traitement et la prévention de la conjonctivite allergique.
